# EUROPEAN PATENT SPECIFICATION

(11) **EP 4 366 808 B1**
(45) Date of publication and mention of the grant of the patent: **04.03.2026**
(21) Application number: 22751781.0
(22) Date of filing: 29.06.2022
(51) Int. Cl.: A61M 16/00, A61M 16/08, A61M 16/20, A63B 23/18, A61M 16/04, A61M 16/10

(54) **RESPIRATORY THERAPY DEVICES**
ATEMTHERAPIEVORRICHTUNGEN
DISPOSITIFS DE THÉRAPIE RESPIRATOIRE

(30) Priority: 06.07.2021 GB 202109719
(43) Date of publication of application: 15.05.2024
(73) Proprietor: ICU Medical International Limited, Lower Pemberton Ashford, Kent TN25 4BF (GB)
(72) Inventor: MARONE-HITZ, Ombeline, Ashford, Kent TN23 3JJ (GB); PERKINS, Jamie Daniel, Ashford, Kent TN25 4BF (GB); TUPPER, Steven Mark, Hythe, Kent CT21 5XW (GB); FIELD, Stephen James, Canterbury, Kent CT4 5LA (GB); KHASAWNEH, Mohammad Qassim Mohammad, Canterbury, Kent CT1 2FP (GB)
(74) Representative: Flint, Jonathan McNeill
(86) International application number: PCT/GB2022/000067
(87) International publication number: WO 2023/281235

(56) References cited:
- EP-A2- 1 103 287
- WO-A2-2009/070851
- CN-A- 111 658 923
- US-B2- 8 267 090

## Description

This invention relates to respiratory therapy devices of the kind having an inlet through which a user breathes, an opening to atmosphere, and a mechanism driven by breathing through the device to produce an oscillating resistance to breathing through the device.

Positive expiratory pressure (PEP) devices, that is, devices that present a resistance to expiration through the device, are now widely used to help treat patients suffering from a range of respiratory impairments, such as chronic obstructive pulmonary disease, bronchitis, cystic fibrosis, and atelectasis. More recently, devices that provide an alternating resistance to flow have been found to be particularly effective in that the expiration and vibration combine to stimulate upwards movement of secretions. One example of such devices is sold under the trade mark Acapella (a registered trade mark of Smiths Medical) by Smiths Medical and is described in US6581598, US6776159, US7059324 and US7699054. Other vibratory respiratory therapy devices are available, such as "Quake" manufactured by Thayer, "AeroPEP" manufactured by Monaghan, "TheraPEP" manufactured by Smiths Medical and "IPV Percussionator" manufactured by Percussionaire Corp. Alternative devices such as "CoughAssist" manufactured by Philips is also available. Respiratory therapy apparatus can instead provide an alternating resistance to flow during inhalation. CN111658923 describes a therapy device with a plate that is moved by inhalation or exhalation. US8267090 describes an inhalation therapy device having a bent inlet hose that oscillates within an outer pipe. WO2009/070851 describes an air filter held in the mouth.

During use, the patient inhales and exhales by mouth through the therapy device, causing the natural warming and moistening carried out by the nose to be bypassed. As a result of this patients can suffer from dryness in their airways. Another perceived problem with such therapy devices is that the forceful exhalation through the device could cause particles from the airway to be driven out of the device and increase the risk of contamination of nearby people and surfaces by bacteria or viruses.

It is an object of the present invention to provide an alternative respiratory therapy device.

According to one aspect of the present invention there is provided a respiratory therapy device according to claim 1. Preferred embodiments are object of the dependent claims.

The gas treatment arrangement is preferably located such that both exhaled and inhaled air passes through the gas treatment arrangement. The gas treatment arrangement may include a filter. The filter is preferably an electrostatic filter. The gas treatment device may additionally or alternatively include a heat and moisture exchange element.

The gas treatment arrangement may be located between the mechanism and the opening to atmosphere such that exhaled air flows to the gas treatment arrangement after flowing through the mechanism.

Alternatively, the gas treatment arrangement may be located at the inlet of the device, such as in a removable mouthpiece.

A respiratory therapy device according to the present invention will now be described, by way of example, with reference to the accompanying drawings, in which:
- Figure 1: is a simplified side elevation view of a first form of the device with its cover raised; and
- Figure 2: is a simplified side elevation view of a second form of device with its cover raised.

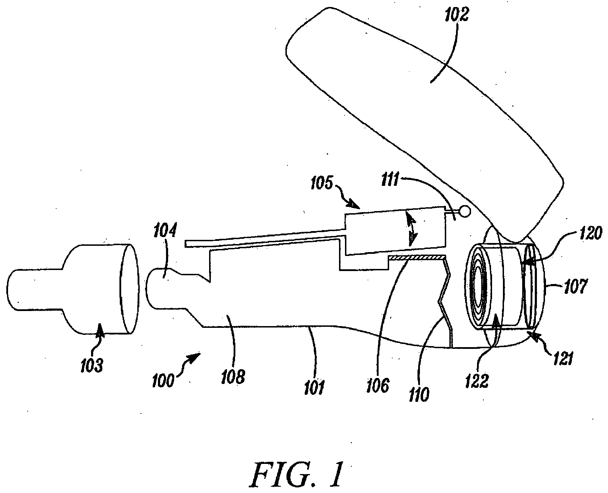

With reference first to Figure 1, the device 100 includes a housing 101 with a normally closed hinged cover 102 and a removable mouthpiece 103. The housing 101 has a patient inlet 104 at its lefthand end to which the mouthpiece 103 is fitted. The housing 101 contains a mechanism for producing an oscillating resistance to expiratory flow in the form of a magnetic rocker system 105 of the kind described in more detail in US6581598. A detailed understanding of the rocker system 105 is not needed to understand the present invention. The rocker system 105 normally closes a valve seat 106 opening from an air-flow passage 108 extending from the patient inlet 104 to an opening or outlet 107 at the opposite end of the housing 101. The air-flow passage 108 includes a one-way valve 110, such as a duck-bill valve, or flap valve, or some other conventional valve that allows flow on one direction. In particular, the valve 110 allows flow from the outlet 107 to the inlet 104 (during inhalation) but prevents or restricts flow in the opposite direction (during expiration). The valve 106 in the rocker system 105 opens from the air-flow passage 108 into a space 111 above the passage that is normally enclosed, when the cover 102 is in the lower, closed position during use. This space 111 communicates with the outlet 107.

It can be seen, therefore, that when the user inhales via the mouthpiece 103 and opening 104 air is drawn through the outlet 107 and the one-way valve 1 10 into the passage 108. The reduced pressure this creates in the passage 108 applies a pressure tending to draw the rocker system 105 further down against the valve seat 106, thereby further enhancing the normally closed state of this valve. When the user exhales through the mouthpiece 103 and inlet 104 this creates an increased pressure in the passage 108, which is prevented from flowing directly to the outlet 107 by the one-way valve 110. Instead, the pressure causes the valve 106 in the magnetic rocker system 105 to open, momentarily lifting the rocker arm and then allowing the pressure below the valve to drop. This allows the rocker arm to fall and close the valve seat 106 again until pressure builds up sufficiently to lift the rocker arm. In this way, the valve 106 alternately opens and closes, causing an oscillation or vibration in the air flow along the passage 108. This oscillation communicates with the user's respiratory system to produce a therapeutic vibration that helps loosen secretions.

As so far described the device 100 is substantially conventional. The device 100, however, is modified over conventional oscillatory therapy devices by the addition of a gas-treatment arrangement 120 disposed in the device in at least the expiratory flow path through the device. In the device shown the gas-treatment arrangement 120 is connected in both the inspiratory and expiratory flow paths between the outlet 107 and the one-way valve 110 (in the inspiratory path) and between the outlet and the rocker system valve 106 (in the expiratory flow path). The gas treatment arrangement 120 is located in the outlet 107 where its inner side is exposed to both air flowing out of the rocker valve 106 through the cavity 111 and where its inner side can allow air to flow through the gas-treatment arrangement 120 to the one-way valve 110. The gas treatment arrangement 120 could be either a filter or an HME element, or, as shown, the combination of both a filter 121 and an HME element 122. The filter 121 is preferably located on the external side of the HME 122 so that it filters exhaled air passing through the HME element and it filters, inhaled air before it passes to the HME element. The filter 121 is preferably an electrostatic filter capable of trapping viruses and bacteria so that any contamination in breath exhaled by the user is filtered and the risk of spreading contamination or infection is reduced. Similarly, the filter 121 protects the user and the device 100 from external contamination when the user inhales. The HME element 122 may be of any conventional kind such as including a coil of corrugated paper treated with a hygroscopic salt, or a similarly treated foam disc. The HME element 122 receives all the air exhaled by the user so is warmed and moistened by passage of this air through the element. Similarly, all the air inhaled by the user through the device 100 must pass through the HME element 122 in the opposite direction so this is warmed and moistened by the heat and moisture stored in the element from the exhaled flow. In this way the drying effect on the user's during use of the device 100 is reduced.

In the device illustrated in Figure 1 the filter 121 and HME element 122 are separate from one another and can be removed from the device 100 and replaced independently if the filter needs to be replaced at different intervals from the HME. Alternatively, the filter could be bonded with one or other of the faces of the HME to form a single unit that is removed and replaced together.

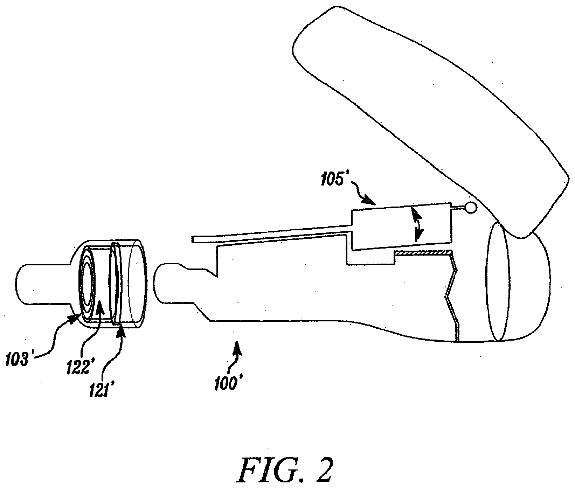

Instead of the HME and filter being mounted in the device at its outlet they could be mounted at the patient inlet, as shown in Figure 2. A convenient way of doing this is to mount the HME 122' and filter 121' in the removable mouthpiece 103'. In the device shown in Figure 2 the filter 121' is located on the downstream side of the HME element 122' during exhalation, that is, closer to the rocker mechanism 105'. This arrangement has the advantage of not requiring any change to the interior of the therapy device 100' to accommodate the HME 122' and filter 121' since these are both incorporated in the mouthpiece 103'. It also makes it easier to replace the HME and filter. This arrangement, by reducing the moisture content of the exhaled breath flowing through the valve mechanism, could reduce contamination of the valve mechanism and enable the device to be used longer before it has to be replaced.

The two arrangements described above have a gas-treatment arrangement including both an HME and a filter. However, it would be possible to use just an HME element, where filtering was not required, or just a filter, where humidification was not required. Where the gas-treatment arrangement includes a filter but not an HME element it could be located in a gas-flow path that receives only expiratory gas flow, with inspiratory flow by-passing the filter. Other forms of gas treatment arrangement could be used.

## Claims

1. A respiratory therapy device (100, 100') having a housing (101), a patient inlet (104) through which a user breathes, an opening (107) to atmosphere, and a mechanism (105, 105' 106) driven by breathing through the device to produce an oscillating resistance to breathing through the device, the device includes a gas treatment arrangement (120, 103') located in line with the mechanism (105, 105', 106) such that at least air exhaled by the patient passes through the gas treatment arrangement before flowing to atmosphere,
**characterized in that** the mechanism that produces an oscillating resistance to breathing includes a rocker arm (105,105') arranged to open and close a valve seat (106),
said valve seat opening from an air-flow passage (108) extending from the patient inlet (104) to the opening (107) at the opposite end of the housing (101), wherein the rocker arm normally closes the valve seat.

2. A device according to Claim 1, wherein the gas treatment arrangement (120, 103') is located such that both exhaled and inhaled air passes through the gas treatment arrangement.

3. A device according to Claim 1 or 2, wherein the gas treatment arrangement (120, 103') includes a filter (121, 121').

4. A device according to Claim 3, wherein the filter is an electrostatic filter (121, 121').

5. A device according to any one of the preceding claims, wherein the gas treatment arrangement (120, 103') includes a heat and moisture exchange element (122, 122').

6. A device according to any one of the preceding claims, wherein the gas treatment arrangement (120) is located between the mechanism (105, 106) and the opening (107) to atmosphere such that exhaled air flows to the gas treatment arrangement (120) after flowing through the mechanism (105, 106).

7. A device according to any one of Claims 1 to 5, wherein the gas treatment arrangement (103') is located at the inlet of the device (100').

8. A device according to Claim 7, wherein the gas treatment arrangement is located in a removable mouthpiece (103').

## Patentansprüche

1. Atemtherapiegerät (100, 100') mit einem Gehäuse (101), einem Patienteneinlass (104), durch den ein Benutzer atmet, einer Öffnung (107) zur Atmosphäre und einem Mechanismus (105, 105', 106), der durch das Atmen durch das Gerät angetrieben wird, um einen oszillierenden Widerstand gegen das Atmen durch das Gerät zu erzeugen, wobei das Gerät eine Gasbehandlungsanordnung (120, 103') beinhaltet, die derart mit dem Mechanismus (105, 105', 106) ausgerichtet ist, dass zumindest vom Patienten ausgeatmete Luft durch die Gasbehandlungsanordnung strömt, bevor sie in die Atmosphäre gelangt,
**dadurch gekennzeichnet, dass** der Mechanismus, der einen oszillierenden Widerstand gegen die Atmung erzeugt, einen Kipphebel (105, 105') zum Öffnen und Schließen eines Ventilsitzes (106) umfasst, wobei sich der Ventilsitz von einem sich vom Patienteneinlass (104) zur Öffnung (107) am gegenüberliegenden Ende des Gehäuses (101) erstreckenden Luftströmungskanal (108) öffnet, wobei der Kipphebel normalerweise den Ventilsitz schließt.

2. Vorrichtung nach Anspruch 1, wobei die Gasbehandlungsanordnung (120, 103') so angeordnet ist, dass sowohl ausgeatmete als auch eingeatmete Luft durch die Gasbehandlungsanordnung strömt.

3. Vorrichtung nach Anspruch 1 oder 2, wobei die Gasbehandlungsanordnung (120, 103') einen Filter (121, 121') umfasst.

4. Vorrichtung nach Anspruch 3, wobei der Filter ein elektrostatischer Filter (121, 121') ist.

5. Vorrichtung nach einem der vorhergehenden Ansprüche, wobei die Gasbehandlungsanordnung (120, 103') ein Wärme- und Feuchtigkeitsaustauschelement (122, 122') umfasst.

6. Vorrichtung nach einem der vorhergehenden Ansprüche, wobei die Gasbehandlungsanordnung (120) zwischen dem Mechanismus (105, 106) und der Öffnung (107) zur Atmosphäre derart angeordnet ist, dass die ausgeatmete Luft nach dem Durchströmen des Mechanismus (105, 106) zur Gasbehandlungsanordnung (120) strömt.

7. Vorrichtung nach einem der Ansprüche 1 bis 5, wobei die Gasbehandlungsanordnung (103') am Einlass der Vorrichtung (100') angeordnet ist.

8. Vorrichtung nach Anspruch 7, wobei sich die Gasbehandlungsanordnung in einem entfernbar Mundstück (103') angeordnet ist.

## Revendications

1. Dispositif de thérapie respiratoire (100, 100') comportant un boîtier (101), une entrée patient (104) par laquelle l'utilisateur respire, une ouverture (107) vers l'atmosphère, et un mécanisme (105, 105', 106) actionné par la respiration à travers le dispositif pour produire une résistance oscillante à la respiration à travers le dispositif,
le dispositif comprend un système de traitement des gaz (120, 103') situé en ligne avec le mécanisme (105, 105', 106) de sorte qu'au moins l'air expiré par le patient passe à travers le système de traitement des gaz avant de s'écouler dans l'atmosphère,
**caractérisé en ce que** le mécanisme qui produit une résistance oscillante à la respiration comprend un bras de bascule (105, 105') disposé pour ouvrir et fermer un siège de soupape (106), ledit siège de soupape s'ouvrant à partir d'un passage d'écoulement d'air (108) s'étendant de l'entrée patient (104) à l'ouverture (107) à l'extrémité opposée du boîtier (101), dans lequel le bras de bascule ferme normalement le siège de soupape.

2. Dispositif selon la revendication 1, dans lequel le dispositif de traitement des gaz (120, 103') est situé de telle sorte que l'air expiré et l'air inhalé passent à travers le dispositif de traitement des gaz.

3. Dispositif selon la revendication 1 ou 2, dans lequel le dispositif de traitement des gaz (120, 103') comprend un filtre (121, 121').

4. Dispositif selon la revendication 3, dans lequel le filtre est un filtre électrostatique (121, 121').

5. Dispositif selon l'une quelconque des revendications précédentes, dans lequel le dispositif de traitement des gaz (120, 103') comprend un élément d'échange de chaleur et d'humidité (122, 122').

6. Dispositif selon l'une quelconque des revendications précédentes, dans lequel le dispositif de traitement des gaz (120) est situé entre le mécanisme (105, 106) et l'ouverture (107) vers l'atmosphère de sorte que l'air expiré s'écoule vers le dispositif de traitement des gaz (120) après avoir traversé le mécanisme (105, 106).

7. Dispositif selon l'une quelconque des revendications 1 à 5, dans lequel le dispositif de traitement des gaz (103') est situé à l'entrée du dispositif (100').

8. Dispositif selon la revendication 7, dans lequel le dispositif de traitement des gaz est situé dans un embout buccal amovible (103').
